# EUROPEAN PATENT SPECIFICATION

(11) **EP 3 398 960 B1**
(45) Date of publication and mention of the grant of the patent: **13.05.2020**
(21) Application number: 16880981.2
(22) Date of filing: 16.12.2016
(51) Int. Cl.: C07K 14/605, C07K 1/06

(54) **METHOD FOR PREPARING SEMAGLUTIDE**
VERFAHREN ZUR HERSTELLUNG VON SEMAGLUTID
PROCÉDÉ DE PRÉPARATION DU SEMAGLUTIDE

(30) Priority: 30.12.2015 CN 201511027176
(43) Date of publication of application: 07.11.2018
(73) Proprietor: Hybio Pharmaceutical Co., Ltd., Guangdong 518057 (CN)
(72) Inventor: CHEN, Xinliang, Shenzhen Guangdong 518057 (CN); MI, Pengcheng, Shenzhen Guangdong 518057 (CN); TAO, Anjin, Shenzhen Guangdong 518057 (CN); YUAN, Jiancheng, Shenzhen Guangdong 518057 (CN)
(74) Representative: HGF Limited
(86) International application number: PCT/CN2016/110373
(87) International publication number: WO 2017/114191

(56) References cited:
- WO-A1-2014/202727
- CN-A- 101 133 082
- CN-A- 103 848 910
- CN-A- 103 848 910
- CN-A- 104 055 735
- CN-A- 104 356 224
- CN-A- 104 356 224
- CN-A- 105 126 082
- LAU JESPER ET AL: "Discovery of the Once-Weekly Glucagon-Like Peptide-1 (GLP-1) Analogue Semaglutide", JOURNAL OF MEDICINAL CHEMISTRY,, vol. 58, no. 18, 1 September 2015 (2015-09-01), pages 7370-7380, XP002764741,
- BABAK BEHNAM AZAD ET AL: "Synthesis and Evaluation of Optical and PET GLP-1 Peptide Analogues for GLP-1R Imaging", MOLECULAR IMAGING, vol. 14, no. 1, 27 January 2015 (2015-01-27), pages 1-16, XP055602268, ISSN: 1536-0121, DOI: 10.2310/7290.2014.00057
- LIN, XUAN et al.: "Advance in Study of Long-Acting Glucagon-Like Peptide-1 Receptor Agonists", CHINESE JOURNAL OF NEW DRUGS, vol. 24, no. 15, 15 August 2015 (2015-08-15), pages 1735-1741, XP009511370,

## Description

### FIELD

The present invention relates to the technical field of polypeptides, and in particular, to a method for preparing Semaglutide.

### BACKGROUND

Semaglutide has a chemical structure of Formula I:

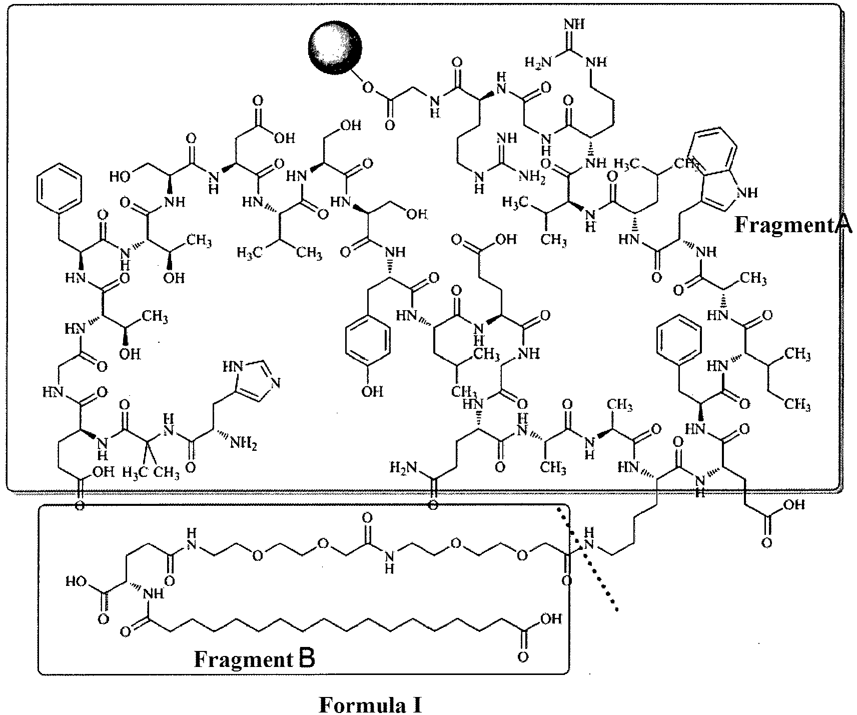

Semaglutide is a long-acting GLP-1 analog developed by Novo Nordisk, which only requires subcutaneous injection once a week and is still in clinical phase III currently. As can be seen from the structure of Semaglutide, a PEG, glutamic acid, and octadecanoic acid aliphatic chain are to be grafted to Lys at position 26 and an unnatural amino acid aminoisobutyric acid is located at position 8 thereof. As compared with Liraglutide, Semaglutide has a longer aliphatic chain and increased hydrophobicity, but a largely enhanced hydrophilicity by short-chain PEG modification. By PEG modification, not only albumin is closely bound and the hydrolysis site for DPP-4 enzyme is masked, but also renal excretion is reduced, biological half-life is prolonged and a long circulation effect is achieved. The peptide sequence (SEQ ID NO:1) has a structure of: 
H-His⁷-Aib⁸-Glu⁹-Gly¹⁰-Thr¹¹-Phe¹²-Thr¹³-Ser¹⁴-Asp¹⁵-Val¹⁶-Ser¹⁷-Ser¹⁸-Ty
r¹⁹-Leu²⁰-Glu²¹-Gly²²-Gln²³-Ala²⁴-Ala²⁵-Lys²⁶（PEG-PEG-γ-Glu- Octadeca
-nedioic Acid ）-Glu²⁷-Phe²⁸-Ile²⁹-Ala³⁰-Trp³¹-Leu³²-Val³³-Arg³⁴-Gly³⁵
-Arg³⁶-Gly³⁷-OH
 H-His⁷-Aib⁸-Glu⁹-Gly¹⁰-Thr¹¹-Phe¹²-Thr¹³-Ser¹⁴-Asp¹⁵-Val¹⁶-Ser¹⁷-Ser¹⁸-Ty r¹⁹-Leu²⁰-Glu²¹-Gly²²-Gln²³-Ala²⁴-Ala²⁸-Lys¹⁶(PEG-PEG-γ-Glu- Octadeca -nedioic Acid)-Glu²⁷-Phe²⁸-Ile²⁹-Ala³⁰-Trp³¹-Leu³²-Val³³-Arg³⁴-Gly³⁵ -Arg³⁶-Gly³⁷-OH

In the prior art (e.g. CN 103848910-A, WO 2014/202727, or CN 104356224-A), a method for synthetizing Semaglutide by sequential coupling using Fmoc solid-phase synthesis to obtain the target polypeptide molecule is provided. In this patent, it is indicated that Lys at position 26 is orthogonally protected with protecting groups Dde, ivDde, Mmt and Mtt, and then sequential coupling is performed on the side chain thereof. It is mentioned in the patent that a pseudoproline dipeptide can be selected for the substitution of Ser and Thr.

The prior art also describes a synthesis method using Fmoc solid phase sequential one by one coupling, wherein Mmt, Mtt, Dde, or ivDde protecting group is selected for temporary orthogonal protection when an amino acid is coupled to the side chain of the lysine, and then a certain method is selected for removing the protecting groups, allowing to couple on the side chain in sequence. The resin is cleaved with a cleaving agent and crude peptides are obtained by precipitating with diethyl ether, and crude peptides are obtained by RP-HPLC.

The currently reported methods all have some deficiencies when Lys is protected with Mmt, Mtt, Dde, or ivDde. It is required in the deprotection of Lys protected with Mmt and Mtt protecting groups that a weakly acidic TFA solution is used and a trapping agent is added, which have to be repeated many times. It is mentioned in the patent that the treatment is required to be repeated 6-12 times for 5-10 min each time in the experiment for removing Mmt and Mtt. Many repeated procedures may affect other protecting agents that are susceptible to acid, such as Trt, tBu, Boc, etc., and may result in side reactions. Mmt and Mtt protecting groups are more difficult to remove, and therefore it is more difficult to guarantee the complete removal of protecting groups during scale-up production. Moreover, 6-12 repeated procedures make the experimental process cumbersome. When 2-CTC (2-trityl chloride resin) is used as a reaction carrier, a weak acid is used, which very easily causes the linker to fall off and the polypeptide fragment to be cleaved, leading to a greatly reduced final yield, an increase in production cost and a decrease in economic efficiency. The deprotection of Lys protected with Dde or ivDde protecting groups requires the use of a hydrazine solution, which has a strong reductibility, and easily undergoes a side reaction with an oxidizing group. Moreover, hydrazine is active in nature, and therefore risky in storage and transportation, and potentially unsafe in the process of scale-up production.

### SUMMARY

In view of this, the present invention provides a method for preparing Semaglutide. In the present invention, a protected amino acid, Fmoc-Lys(Alloc)-OH is used as a raw material, and Pd(PPh₃)₄ is selected for deprotection. On one hand, the operation process is simple, and only 1-2 removal reactions for 10-30 min each time are simply required, thus producing no side reaction, making it safe to operate, and making it convenient for scale-up production. In the process, Boc-His(Boc)-OH.DCHA and Boc-His(Trt)-OH are used as raw materials, which can minimize the risk of His racemization. Moreover, the synthesis efficiency is increased by using a special fragment for coupling.

In order to achieve the above objects of the present invention, the following technical solutions are provided:

The present invention provides a preparation method of Semaglutide, comprising the steps of:
step 1: coupling Gly to a resin by solid phase synthesis to obtain Gly-resin; and
step 2: successively coupling the Gly-resin prepared in step 1 to an amino acid according to the sequence of the Semaglutide by sequential coupling; cleaving, and purifying;
   wherein Fmoc-Lys(Alloc)-OH is used as a raw material of Lys²⁶;
   H-His⁷ is synthesized using Boc-His(Trt)-OH or Boc-His(Boc)-OH.DCHA as a raw material; and
   the Lys²⁶ side chain is prepared by sequential coupling or fragmental synthesis.

In some particular embodiments of the present invention, the fully-protected fragment of Fmoc-PEG-PEG-γ-Glu-octadecanedioic acid used in the fragmental synthesis of the Lys²⁶ side chain in the preparation method is prepared by successively coupling a chloride resin to Fmoc-PEG-OH, Fmoc-PEG-OH, Fmoc-Glu(OH)-OtBu and Octadecanedioic Acid mono-tert-butyl ester, cleaving, and recrystallizing to obtain PEG-PEG-γ-Glu-Octadecanedioic Acid mono-tert-butyl ester.

In some particular embodiments of the present invention, the resin in step 1 of the preparation method is chosen from wang resin or 2-CTC resin.

The wang resin has a degree of substitution of 0.1-0.5 mmol/g, preferably 0.25-0.35 mmol/g.

The 2-CTC resin has a degree of substitution of 0.2-0.6 mmol/g, preferably 0.25-0.45 mmol/g.

The wang resin is coupled by DIC+A+DMAP, wherein A is HOBt or HOAt.

The 2-CTC resin is coupled by DiPEA method.

In some particular embodiments of the present invention, the coupling in step 2 of the preparation method is performed by condensing with DIC+A or B+A+C until the resin is detected to be transparent with ninhydrin, wherein A is chosen from HOBt or HOAt, B is chosen from HBTU, HATU, TBTU, PyAOP, or PyBOP, C is chosen from DIPEA or TMP; and the solvent is chosen from one or a mixture of two or more of DMF, CH₂Cl₂, NMP, or DMSO.

In some particular embodiments of the present invention, the coupling in the fragmental synthesis of the Lys²⁶ side chain in the preparation method is performed by condensing with DIC+A or B+A+C until the resin is detected to be transparent with ninhydrin, wherein A is chosen from HOBt or HOAt, B is chosen from HBTU, HATU, TBTU, PyAOP, or PyBOP, C is chosen from DIPEA or TMP; and the solvent is chosen from one or a mixture of two or more of DMF, CH₂Cl₂, NMP, or DMSO.

In some particular embodiments of the present invention, the cleaving in the fragmental synthesis of the Lys²⁶ side chain in the preparation method is performed with 1-2% TFA/CH₂Cl₂ (V/V) or 20% TFE/CH₂Cl₂ (V/V).

In some particular embodiments of the present invention, the recrystallizing in the fragmental synthesis of the Lys²⁶ side chain in the preparation method is performed with one or a mixture of two or more of Et₂O, CH₂Cl₂, CHCl₃, MeOH, CH₃CN, THF, EtOAc, EtOH, Toluene, Hexane, or Acetone, preferably a mixed solvent of MeOH and CH₂Cl₂.

In some particular embodiments of the present invention, Alloc in the Fmoc-Lys(Alloc)-OH in the preparation method is removed twice with 5-10 equivalents of morpholine (or 5-10 equivalents of phenylsilane as an alternative thereof) and 0.1-0.3 equivalents of Pd(PPh₃)₄ for 10-30 min each time, wherein CH₂Cl₂ is used as a solvent in the removal.

In some particular embodiments of the present invention, in the preparation method, for Boc-His(Trt)-OH, the coupling is performed by a B+A+C system; and for Boc-His(Boc)-OH.DCHA, the coupling is performed by a HOAT + HATU + TMP system;
wherein A is chosen from HOBt or HOAt, B is chosen from HBTU, HATU, TBTU, PyAOP or PyBOP, C is chosen from DIPEA or TMP; and the solvent is chosen from one or a mixture of two or more of DMF, CH₂Cl₂, NMP or DMSO.

In some particular embodiments of the present invention, the cleaving in step 2 of the preparation method is performed with an reagent, comprising one or a mixture of two or more of TFA, PhSMe, PhOMe, EDT, H₂O, TIS or PhOH;
wherein TFA, PhSMe, PhOMe, EDT, H₂O, TIS and PhOH are in a volume ratio of 80-90:0-5:0-3:0-5:0-5:0-2:0-5, with TFA:PhSMe:PhOH:EDT:H₂O = 85:5:3:5:2 being preferred.

In the present invention, a protected amino acid, Fmoc-Lys(Alloc)-OH is used as a raw material, and Pd(PPh₃)₄ is selected for deprotection. On one hand, the operation process is simple, and only 1-2 removal reactions for 10-30 min each time are simply required, thus producing no side reaction, making it safe to operate, and making it convenient for scale-up production. In the process, Boc-His(Boc)-OH.DCHA and Boc-His(Trt)-OH are used as raw materials, which can minimize the risk of His racemization. Moreover, the synthesis efficiency is increased by using a special fragment for coupling.

In the present invention, Fmoc-Lys(Alloc)-OH is used as a raw material, which simplifies the deprotection step, and eliminates the side reactions brought out during the deprotection of Mmt and Mtt, and the operation safety risk in removing Dde or ivDde protecting groups in the scale-up process.

His racemization impurities are reduced by using the coupling system of Boc-His(Trt)-OH and Boc-His(Boc)-OH.DCHA in the present invention.

In the present invention, an acidic TFA solution is to be used to remove Mmt and Mtt protecting groups 6-12 times, which increases the complexity of the operation. Moreover, the acidic solution will corrode the equipment and increase the production cost. The acidic solution may lead to the leaving of protecting groups easily removed under weakly acidic conditions such as tBu, Boc, increasing the possibility of side reactions. When Mmt and Mtt protecting groups are used in the synthesis of Semaglutide, only acid-resistant Wang resin can be used, rather than less acid-resistant chlorine resin, limiting the range of resins used.

In the present invention, a hydrazine solution is to be used in the removal of the Dde or ivDde protecting group, and there is a large potential safety hazard when a large amount of hydrazine is used in the scale-up production.

By using a side chain fragment in the present invention, the efficiency of synthesis is increased, the coupling synthesis steps are effectively reduced, and the risk of increased impurities due to the increased number of coupling is reduced.

The beneficial effects of the present invention include the following.
1. Fmoc-Lys(Alloc)-OH, Boc-His(Trt)-OH and Boc-His(Boc)-OH.DCHA as raw materials are used for the respective amino acid coupling.
2. A specific coupling method of Boc-His(Boc)-OH.DCHA or Boc-His(Trt)-OH, HOAt/HATU/TMP, HOBt/HBTU/DiPEA or HOBt/PYBOP/DiPEA is adopted to reduce the risk of histidine racemization.
3. The use of a side chain fragment enhances synthesis efficiency. When sequential coupling is used, the synthesis yield of the crude peptide is 83%, the purity of the crude peptide is 65.3%, the purification yield is 23.6% and the purity of the refined peptide is 99.02%. When fragmental coupling is used, the synthesis yield of the crude peptide is 93%, the purity of the crude peptide is 72.4%, the purification yield is 34.4% and the purity of the refined peptide is 99.60%. (See Examples 17 and 18).

### BRIEF DESCRIPTION OF THE DRAWINGS

In order to more clearly illustrate the technical solutions in Examples of the present invention or in the prior art, the drawings to be used in the description of Examples or the prior art will be briefly described below.

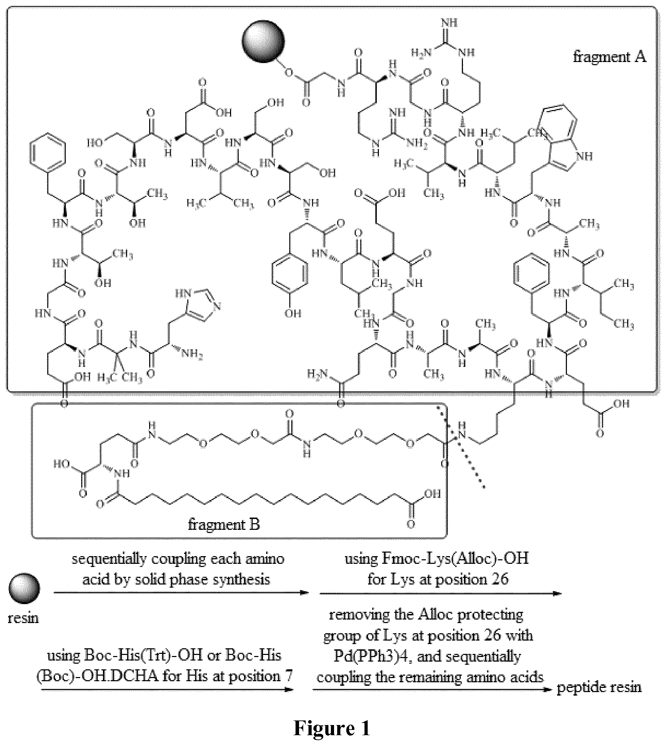

Figure 1 shows the structure and preparation process of Semaglutide;
Figure 2 shows the detection results of His racemization impurities produced by His coupling during the preparation of Semaglutide in Example 9 and the prior art (Method 1);
Figure 3 shows the HPLC detection spectrum of His racemization impurities produced by His coupling during the preparation of Semaglutide in Example 10;
Figure 4 shows the MALDI-TOF mass spectrum of Semaglutide prepared in Example 16;
Figure 5 shows the HPLC spectrum of Semaglutide prepared in Example 18;
Figure 6 shows the MALDI-TOF mass spectrum of Semaglutide prepared in Example 15;
Figure 7 shows the MALDI-TOF mass spectrum of Semaglutide prepared in Example 17; and
Figure 8 shows the MALDI-TOF mass spectrum of Semaglutide prepared in Example 18.

### DETAILED DESCRIPTION OF EMBODIMENTS

The present invention discloses a method for preparing Semaglutide, which can be achieved by those skilled in the art by properly improving process parameters in light of the disclosure. In particular, it should be noted that all similar substitutions and modifications will be apparent to those skilled in the art. The method and application of the present invention have been described by preferred examples thereof, and it is apparent that related individuals can make modifications or proper changes and combination to the methods and applications described herein so as to achieve and apply the technology of the present application.

The present invention describes two methods for the synthesis of Semaglutide:

### Method 1: Sequential coupling

The route for sequential coupling is:

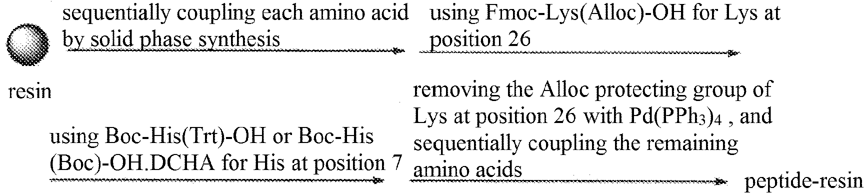

### Method 2: Sequential coupling + fragmental synthesis

The schematic diagram for analysis of sequential coupling + fragmental synthesis is:

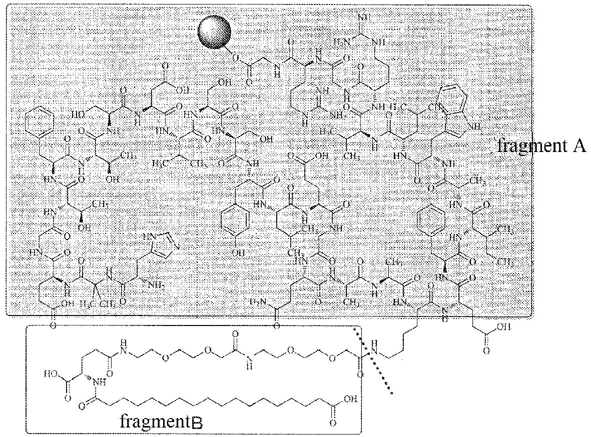

The synthesis route of the present invention comprises the following steps:
1) selecting an appropriate carrier and attaching Fmoc-Gly-OH to the resin by solid phase synthesis;
2) successively coupling each amino acid by sequential coupling;
3) preparing a fully-protected fragment of Fmoc-PEG-PEG-γ-Glu-Octadecanedioic Acid using a chloride resin and purifying by recrystallization;
4) using Fmoc-Lys(Alloc)-OH as a raw material of Lys²⁶, with deprotection under the condition of Pd (PPh₃)₄ and morpholine;
5) synthesizing H-His⁷ with Boc-His(Trt)-OH or Boc-His(Boc)-OH.DCHA as a raw material;
6) coupling Octadecanedioic Acid using octadecanedioic acid mono-tert-butyl ester as a raw material;
7) cleaving the peptide resin; and
8) purifying the crude peptide.

The resin to which Fmoc-Gly-OH is attached in step 1) can be chosen from wang resin or 2-CTC resin.

In step 1), the Fmoc-Gly-wang resin has a degree of substitution within a range of 0.1-0.5 mmol/g, preferably 0.25-0.35 mmol/g when used in the synthesis; and the Fmoc-Gly-2-CTC resin has a degree of substitution within a range of 0.2-0.6 mmol/g, preferably 0.25-0.45 mmol/g when used in the synthesis. DIC+A+DMAP is used in the attachment of wang resin, wherein A is HOBt or HOAt; and DiPEA is used in the attachment of 2-CTC chloride resin.

The amino acid or amino acid analogs used in the sequential coupling of step 2) are Fmoc protected amino acids, wherein Boc-His(Trt)-OH or Boc-His(Boc)-OH.DCHA is used for H-His⁷, Fmoc-Lys(Alloc)-OH is used for Lys²⁶, and octadecanedioic acid mono-tert-butyl ester is used for the coupling of Octadecanedioic Acid.

Condensation is performed using DIC+A or B+A+C in the coupling of step 2) until the resin is detected to be transparent with ninhydrin; wherein A is HOBt or HOAt, B is HBTU, HATU, TBTU, PyAOP, or PyBOP, C is DIPEA or TMP, and the solvent is chosen from DMF, CH₂Cl₂, NMP, DMSO or a mixed solvent of any thereof.

In step 3), the fully-protected fragment of PEG-PEG-γ-Glu-Octadecanedioic Acid is prepared using 2-CTC chloride resin, wherein cleavage is performed with 1-2% TFA/CH₂Cl₂ or 20% TFE/CH₂Cl₂; and condensation is performed with DIC+A or B+A+C in the coupling until the resin is detected to be transparent with ninhydrin; wherein A is HOBt or HOAt, B is HBTU, HATU, TBTU, PyAOP, or PyBOP, C is DIPEA or TMP, and the solvent is chosen from DMF, CH₂Cl₂, NMP, DMSO or a mixed solvent of any thereof.

The solvent used in the recrystallization of step 3) is chosen from Et₂O, CH₂Cl₂, CHCl₃, MeOH, CH₃CN, THF, EtOAc, EtOH, Toluene, Hexane, Acetone or a mixed solvent of any thereof, preferably a mixed solvent of MeOH and CH₂Cl₂.

In step 4), Alloc in Fmoc-Lys(Alloc)-OH is removed twice using 0.1-0.3 equivalents of Pd(PPh₃)₄ and 5-10 equivalents of morpholine or phenylsilane for 10-30 min each time, wherein CH₂Cl₂ is used as a solvent in the removal.

In step 5), the B+A+C system is preferred when Boc-His(Trt)-OH is used for coupling; and the HOAT+HATU+TMP system is preferred when Boc-His(Boc)-OH.DCHA is used for coupling.

The cleaving in step 7) is performed with TFA:PhSMe:PhOMe:EDT:H₂O:TIS:PhOH=80∼90:0∼5:0∼3:0∼5:0∼5:0∼2:0∼5(V:V), with TFA:PhSMe:PhOH:EDT:H₂O=85:5:3:5:2 being preferred.

The abbreviations and respective meanings thereof are shown in Table 1 below.

**Table 1: Abbreviations and respective meanings thereof**

| | |
|---|---|
| Fmoc | 9-fluorenylmethoxycarbonyl |
| 2-CTC-Resin | 2-chlorotrityl chloride resin |
| tBu | tert-butyl |
| OtBu | tert-butoxy |
| Alloc | allyloxycarbonyl |
| DCM | dichloromethane |
| DBLK | 20% hexahydropyridine/DMF solution |
| DIPEA | *N,N-*diisopropylethylamine |
| HOBt | 1-hydroxybenzotriazole |
| HOAt | 1-hydroxy-7-azobenzotriazole |
| PyBOP | benzotriazol-1-yl-oxytripyrrolidinyl hexafluorophosphate |
| DMSO | dimethyl sulfoxide |
| HATU | *O*-(7-azobenzotriazole)-N,N,N',N'-tetramethyluronium hexafluorophosphate |
| | |
| HPLC | High Efficiency Liquid Chromatography |
| DMF | *N,N*-dimethylformamide |
| TFA | trifluoroacetic acid |
| TMP | 2,4,6-trimethylpyridine |
| Mmt | 4-methoxytrityl |
| Mtt | 4-methyltrityl |
| DCHA | dicyclohexyl amine |
| Boc | t-butoxycarbonyl |
| Trt | trityl |

The raw materials and reagents used in the preparation method of Semaglutide provided by the present invention are all commercially available.

In the following, the present invention will be further illustrated in combination with Examples:

### Example 1: Preparation of Fmoc-Gly-Wang Resin

210 g of dry Wang Resin (with a degree of substitution of 0.845 mmol/g) was weighed and added into a solid phase reaction column. The resin was firstly washed twice with DMF, again swollen for 30 min with DMF in a volume 2-3 times the resin bed, and washed with DMF three times and DCM twice, for future feeding.

Under cooling in ice bath, 105.48 g of Fmoc-Gly-OH and 47.92 g of HOBt were dissolved in a mixed solvent of DMF and DCM. After the amino acid was dissolved, 55 ml of DIC was slowly added, and the reactant was poured into the reaction column following activation for 3 min. 2.17 g of DMAP was added, and stirred with air-blowing for 2 h. After the reaction was completed, an appropriate amount of a mixed solution of acetic anhydride and pyridine (volume ratio: Ac2O/Pyridine=7/6) was added to block the reaction for 5 h or more, following washing three times with DMF. After the reaction was stopped, Fmoc-Gly-Wang Resin with a degree of substitution of 0.496 mmol/g was obtained, following washing 6 times with DMF, shrinking twice with methanol, and drying under reduced pressure.

### Example 2: Preparation of Fmoc-Gly-Wang Resin

218 g of dry Wang Resin (with a degree of substitution of 0.845 mmol/g) was weighed and added into a solid phase reaction column. The resin was firstly washed twice with DMF, again swollen for 30 min with DMF in a volume 2-3 times the resin bed, and washed with DMF three times and DCM twice, for future feeding.

Under cooling in ice bath, 26.88 g of Fmoc-Gly-OH and 12.03 g of HOBt were dissolved in a mixed solvent of DMF and DCM. After the amino acid was dissolved, 11 ml of DIC was slowly added, and the reactant was poured into the reaction column following activation for 3 min. 1.12 g of DMAP was added, and stirred with air-blowing for 45 min. After the reaction was completed, an appropriate amount of a mixed solution of acetic anhydride and pyridine (volume ratio: Ac2O/Pyridine=7/6) was added to block the reaction overnight, following washing three times with DMF. After the reaction was stopped, Fmoc-Gly-Wang Resin with a degree of substitution of 0.108 mmol/g was obtained, following washing 6 times with DMF, shrinking twice with methanol, and drying under reduced pressure.

### Example 3: Preparation of Fmoc-Gly-Wang Resin

220 g of dry Wang Resin (with a degree of substitution of 0.845 mmol/g) was weighed and added into a solid phase reaction column. The resin was firstly washed twice with DMF, again swollen for 30 min with DMF in a volume 2-3 times the resin bed, and washed with DMF three times and DCM twice, for future feeding.

Under cooling in ice bath, 55.76 g of Fmoc-Gly-OH and 25.11 g of HOBt were dissolved in a mixed solvent of DMF and DCM. After the amino acid was dissolved, 29 ml of DIC was slowly added, and the reactant was poured into the reaction column following activation for 3 min. 2.17 g of DMAP was added, and stirred with air-blowing for 90 min. After the reaction was completed, an appropriate amount of a mixed solution of acetic anhydride and pyridine (volume ratio: Ac2O/Pyridine=7/6) was added to block the reaction overnight, following washing three times with DMF. After the reaction was stopped, Fmoc-Gly-Wang Resin with a degree of substitution of 0.314 mmol/g was obtained, following washing 6 times with DMF, shrinking twice with methanol, and drying under reduced pressure.

### Example 4: Preparation of Fmoc-Gly-2-CTC-Resin

198 g of dry 2-CTC-Resin (with a degree of substitution of 0.568 mmol/g) was weighed and added into a solid phase reaction column. The resin was firstly washed twice with DMF, again swollen for 30 min with DMF in a volume 2-3 times the resin bed, and washed with DMF three times and DCM twice, for future feeding.

Under cooling in ice bath, 64.58 g of Fmoc-Gly-OH and 75 ml of DIPEA were dissolved in a mixed solvent of DMF and DCM. After the amino acid was dissolved, the activated Fmoc-Gly-OH was slowly added into the reaction column for 2 h. After the reaction was completed, a blocking solution consisting of 50 ml methanol and 36 ml DIPEA was added into the reaction column for 30 min. Fmoc-Gly-2-CTC-Resin with a degree of substitution of 0.535 mmol/g was obtained, following washing 3 times with DMF, shrinking twice with methanol, and drying under reduced pressure.

### Example 5: Preparation of Fmoc-Gly-2-CTC-Resin

202 g of dry 2-CTC-Resin (with a degree of substitution of 0.388 mmol/g) was weighed and added into a solid phase reaction column. The resin was firstly washed twice with DMF, again swollen for 30 min with DMF in a volume 2-3 times the resin bed, and washed with DMF three times and DCM twice, for future feeding.

Under cooling in ice bath, 46.75 g of Fmoc-Gly-OH and 54 ml of DIPEA were dissolved in a mixed solvent of DMF and DCM. After the amino acid was dissolved, the activated Fmoc-Gly-OH was slowly added into the reaction column for 2 h. After the reaction was completed, a blocking solution consisting of 48 ml methanol and 26 ml DIPEA was added into the reaction column for another 30 min. Fmoc-Gly-2-CTC-Resin with a degree of substitution of 0.368 mmol/g was obtained, following washing 3 times with DMF, shrinking twice with methanol, and drying under reduced pressure.

### Example 6: Preparation of Fmoc-Gly-2-CTC-Resin

216 g of dry 2-CTC-Resin (with a degree of substitution of 0.388 mmol/g) was weighed and added into a solid phase reaction column. The resin was firstly washed twice with DMF, again swollen for 30 min with DMF in a volume 2-3 times the resin bed, and washed with DMF three times and DCM twice, for future feeding.

Under cooling in ice bath, 25.16 g of Fmoc-Gly-OH and 28 ml of DIPEA were dissolved in a mixed solvent of DMF and DCM. After the amino acid was dissolved, the activated Fmoc-Gly-OH was slowly added into the reaction column for 2 h. After the reaction was completed, a blocking solution consisting of 45 ml methanol and 15 ml DIPEA was added into the reaction column for another 30 min. Fmoc-Gly-2-CTC-Resin with a degree of substitution of 0.211 mmol/g was obtained, following washing 3 times with DMF, shrinking twice with methanol, and drying under reduced pressure.

### Example 7: Cleavage of the peptide resin fragment, PEG-PEG-γ-Glu-Octadecanedioic Acid mono-tert-butyl ester

239 g of dry 2-CTC-Resin (with a degree of substitution of 0.568 mmol/g) was weighed and added into a solid phase reaction column. The resin was firstly washed twice with DMF, again swollen for 30 min with DMF in a volume 2-3 times the resin bed, and washed with DMF three times and DCM twice, for future feeding.

Under cooling in ice bath, 104.6 g of Fmoc-PEG-OH and 94 ml of DIPEA were dissolved in a mixed solvent of DMF and DCM. After the amino acid was dissolved, the activated Fmoc-PEG-OH was slowly added into the reaction column for 2 h. After the reaction was completed, a blocking solution consisting of 65 ml methanol and 47 ml DIPEA was added into the reaction column for another 30 min. 3 washes were performed with DMF.

Fmoc-PEG-OH, Fmoc-Glu(OH)-OtBu and octadecanedioic acid mono-tert-butyl ester were sequentially coupled by conventional amino acid coupling. After the coupling was completed, 345 g of peptide resin was obtained following shrinking with methanol, followed by cleaving with 3.5 L of 1% TFA/CH₂Cl₂ solution for 2 h, and concentrating under reduced pressure to yield about 108 g of fully-protected peptides. 98 g fully-protected peptides were obtained by recrystallization with a mixed solvent of methanol and dichloromethane. The molecular weight by MALDI-TOF mass spectrum is [M+H⁺]=846.8.

### Example 8: Coupling of the peptide resin fragment, PEG-PEG-γ-Glu-Octadecanedioic Acid mono-tert-butyl ester

187 g of dry 2-CTC-Resin (with a degree of substitution of 0.752 mmol/g) was weighed and added into a solid phase reaction column. The resin was firstly washed twice with DMF, again swollen for 30 min with DMF in a volume 2-3 times the resin bed, and washed with DMF three times and DCM twice, for future feeding.

Under cooling in ice bath, 108.4 g of Fmoc-PEG-OH and 98 ml of DIPEA were dissolved in a mixed solvent of DMF and DCM. After the amino acid was dissolved, the activated Fmoc-PEG-OH was slowly added into the reaction column for 2 h. After the reaction was completed, a blocking solution consisting of 60 ml methanol and 50 ml DIPEA was added into the reaction column for another 30 min. 3 washes were performed with DMF.

Fmoc-PEG-OH, Fmoc-Glu(OH)-OtBu and octadecanedioic acid mono-tert-butyl ester were sequentially coupled by conventional amino acid coupling. After the coupling was completed, 310 g of peptide resin was obtained following shrinking with methanol, followed by cleaving with 3.1 L of 20% TFE/CH₂Cl₂ solution for 2 h, and concentrating under reduced pressure to yield about 115 g of fully-protected peptides. 108 g fully-protected peptides were obtained by recrystallization with a mixed solvent of ethyl acetate and trichlormethane. The molecular weight by MALDI-TOF mass spectrum is [M+H⁺]=846.6.

### Example 9: Synthesis of peptide resin of Semaglutide

The Fmoc-Gly-Wang resin synthesized in Example 1 was used for the synthesis of peptide resin of Semaglutide. 202 g Fmoc-Gly-Wang Resin (100 mmol) with a degree of substitution of 0.496 mmol/g was weighed and swelled for 30 min with DMF, followed by sequentially coupling according to the amino acid sequence of Semaglutide with 2-5 equivalents of amino acid feeds and condensing with DIC+A or B+A+C, until the resin was detected to be transparent with ninhydrin, wherein A was HOBt or HOAt; B was HBTU, HATU, TBTU, PyAOP, or PyBOP; C was DIPEA or TMP; and the solvent was chosen from DMF, CH₂Cl₂, NMP, DMSO or a mixed solvent of any thereof; and for Boc-His(Trt)-OH, the coupling was performed by a HOBt/HBTU/DiPEA system.

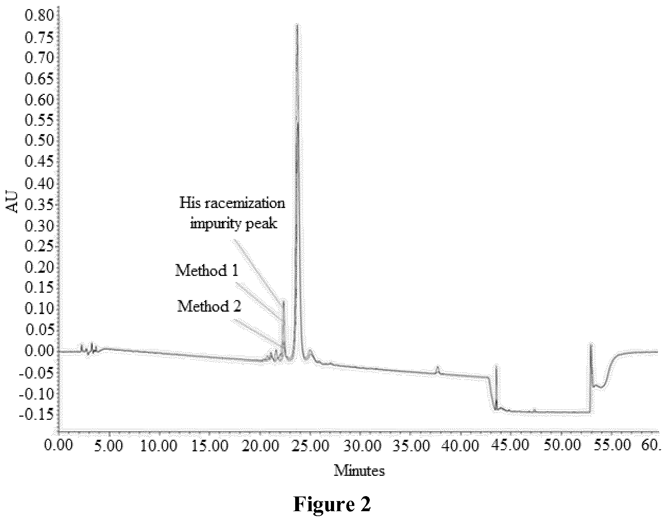

The crude peptide obtained with a Boc-His (Trt)-OH/HOBt/HBTU/DiPEA coupling system was sampled and detected to have a significantly smaller peak of His racemization impurities than the Boc-His (Boc)-OH coupling system (HOBt/HBTU/DiPEA) used in the literature, as shown in Figure 2.
Method 1: Boc-His(Boc)-OH/HOBt/HBTU/DiPEA system, with 2%-3% of His racemization impurities.
Method 2 (the present invention): Boc-His(Trt)-OH/HOBt/HBTU/DiPEA system, with less than 1% of His racemization impurities.

Before the coupling of the Lys side chain, Alloc protecting groups were removed twice with a solution of 0.2 equivalents of Pd(PPh₃)₄ and 10 equivalents of morpholine in methylene chloride for 30 min each time. The remaining amino acids on the side chain were coupled according to a conventional method until transparent, and 826 g peptide resin was obtained after coupling completed.

### Example 10: Synthesis of peptide resin of Semaglutide

The Fmoc-Gly-Wang resin synthesized in Example 2 was used for the synthesis of peptide resin of Semaglutide. 200 g Fmoc-Gly-Wang Resin (21.6 mmol) with a degree of substitution of 0.108 mmol/g was weighed and swelled for 30 min with DMF, followed by sequentially coupling according to the amino acid sequence of Semaglutide with 2-5 equivalents of amino acid feeds and condensing with DIC+A or B+A+C, until the resin was detected to be transparent with ninhydrin, wherein A was HOBt or HOAt; B was HBTU, HATU, TBTU, PyAOP, or PyBOP; C was DIPEA or TMP; and the solvent was chosen from DMF, CH₂Cl₂, NMP, DMSO or a mixed solvent of any thereof; and for Boc-His(Boc)-OH.DCHA, the coupling was performed by a HOAt/HATU/TMP system.

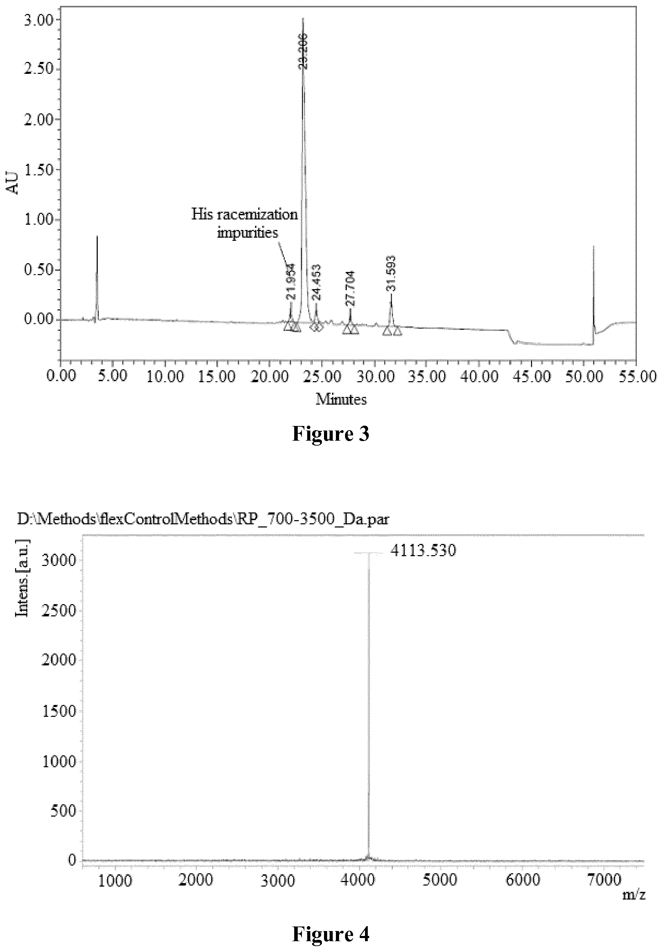

Before the coupling of the Lys side chain, Alloc protecting groups were removed twice with a solution of 0.2 equivalents of Pd(PPh₃)₄ and 10 equivalents of phenylsilane in methylene chloride for 30 min each time. The remaining amino acids on the side chain were coupled according to a conventional method until transparent, and 335 g peptide resin was obtained after coupling completed, as shown in Figure 3.

There was 0.98% His racemization impurities when Boc-His(Boc)-OH.DCHA/HOAt/HATU/TMP system was used.

### Example 11: Synthesis of peptide resin of Semaglutide

The Fmoc-Gly-Wang resin synthesized in Example 3 was used for the synthesis of peptide resin of Semaglutide. 102 g Fmoc-Gly-Wang Resin (32 mmol) with a degree of substitution of 0.314 mmol/g was weighed and swelled for 30 min with DMF, followed by sequentially coupling according to the amino acid sequence of Semaglutide with 2-5 equivalents of amino acid feeds and condensing with DIC+A or B+A+C, until the resin was detected to be transparent with ninhydrin, wherein A was HOBt or HOAt; B was HBTU, HATU, TBTU, PyAOP, or PyBOP; C was DIPEA or TMP; and the solvent was chosen from DMF, CH₂Cl₂, NMP, DMSO or a mixed solvent of any thereof; and for Boc-His(Trt)-OH, the coupling was performed by a HOBt/HBTU/DiPEA or HOBt/PYBOP/DiPEA system.

Before the coupling of the Lys side chain, Alloc protecting groups were removed twice with a solution of 0.2 equivalents of Pd(PPh₃)₄ and 10 equivalents of phenylsilane in methylene chloride for 30 min each time. The coupling of the remaining amino acids on the side chain was completed at one time with the PEG-PEG-γ-Glu-Octadecanedioic Acid mono-tert-butyl ester fragment, and 306 g peptide resin was obtained after coupling completed.

### Example 12: Synthesis of peptide resin of Semaglutide

The Fmoc-Gly-2-CTC resin synthesized in Example 4 was used for the synthesis of peptide resin of Semaglutide. 94 g Fmoc-Gly-2-CTC Resin (50 mmol) with a degree of substitution of 0.535 mmol/g was weighed and swelled for 30 min with DMF, followed by sequentially coupling according to the amino acid sequence of Semaglutide with 2-5 equivalents of amino acid feeds and condensing with DIC+A or B+A+C, until the resin was detected to be transparent with ninhydrin, wherein A was HOBt or HOAt; B was HBTU, HATU, TBTU, PyAOP, or PyBOP; C was DIPEA or TMP; and the solvent was chosen from DMF, CH₂Cl₂, NMP, DMSO or a mixed solvent of any thereof; and for Boc-His(Trt)-OH, the coupling was performed by a HOBt/HBTU/DiPEA or HOBt/PYBOP/DiPEA system.

Before the coupling of the Lys side chain, Alloc protecting groups were removed twice with a solution of 0.2 equivalents of Pd(PPh₃)₄ and 10 equivalents of phenylsilane in methylene chloride for 30 min each time. The remaining amino acids on the side chain were coupled according to a conventional method until transparent, and 396 g peptide resin was obtained after coupling completed.

### Example 13: Synthesis of peptide resin of Semaglutide

The Fmoc-Gly-2-CTC resin synthesized in Example 5 was used for the synthesis of peptide resin of Semaglutide. 114 g Fmoc-Gly-2-CTC Resin (42 mmol) with a degree of substitution of 0.368 mmol/g was weighed and swelled for 30 min with DMF, followed by sequentially coupling according to the amino acid sequence of Semaglutide with 2-5 equivalents of amino acid feeds and condensing with DIC+A or B+A+C, until the resin was detected to be transparent with ninhydrin, wherein A was HOBt or HOAt; B was HBTU, HATU, TBTU, PyAOP, or PyBOP; C was DIPEA or TMP; and the solvent was chosen from DMF, CH₂Cl₂, NMP, DMSO or a mixed solvent of any thereof; and for Boc-His(Boc)-OH.DCHA, the coupling was performed by a HOAt/HATU/TMP system.

Before the coupling of the Lys side chain, Alloc protecting groups were removed twice with a solution of 0.2 equivalents of Pd(PPh₃)₄ and 10 equivalents of morpholine in methylene chloride for 30 min each time. The remaining amino acids on the side chain were coupled according to a conventional method until transparent, and 362 g peptide resin was obtained after coupling completed.

### Example 14: Synthesis of peptide resin of Semaglutide

The Fmoc-Gly-2-CTC resin synthesized in Example 6 was used for the synthesis of peptide resin of Semaglutide. 95 g Fmoc-Gly-2-CTC Resin (20 mmol) with a degree of substitution of 0.211 mmol/g was weighed and swelled for 30 min with DMF, followed by sequentially coupling according to the amino acid sequence of Semaglutide with 2-5 equivalents of amino acid feeds and condensing with DIC+A or B+A+C, until the resin was detected to be transparent with ninhydrin, wherein A was HOBt or HOAt; B was HBTU, HATU, TBTU, PyAOP, or PyBOP; C was DIPEA or TMP; and the solvent was chosen from DMF, CH₂Cl₂, NMP, DMSO or a mixed solvent of any thereof; and for Boc-His(Boc)-OH.DCHA, the coupling was performed by a HOAt/HATU/TMP system.

Before the coupling of the Lys side chain, Alloc protecting groups were removed twice with a solution of 0.2 equivalents of Pd(PPh₃)₄ and 10 equivalents of morpholine in methylene chloride for 30 min each time. The coupling of the remaining amino acids on the side chain was completed at one time with the PEG-PEG-γ-Glu-Octadecanedioic Acid mono-tert-butyl ester fragment, and 223 g peptide resin was obtained after coupling completed.

### Example 15: Cleavage and purification of Semaglutide

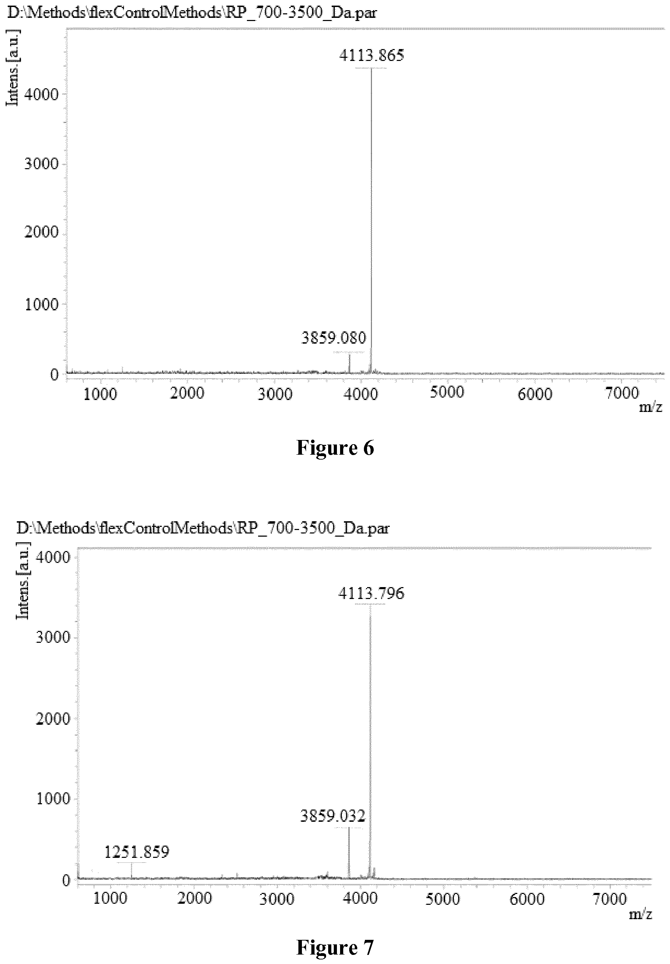

400 g of Semaglutide Wang resin obtained in Example 9 was taken and added into a 5 L reaction kettle. 4 L cleaving agent was formulated in a volume ratio of TFA:thioanisole:anisole:EDT=90:5:3:2, and pre-frozen for 2 h, and then poured into the 5 L reaction kettle, allowing to react for 2 h at the room temperature. After the reaction was completed, the resin was filtered off and the filtrate was collected. The resin was washed with a small amount of TFA, and the filtrates were combined. The filtrate was added to 40 L of ice-cold anhydrous diethyl ether, precipitated, centrifuged, washed with anhydrous diethyl ether, and dried under vacuum to obtain 182 g of crude Semaglutide with a purity of 66.8%. MALDI-TOF: (M+H)⁺=4113.9, as shown in Figure 6.

42.6 g of crude Semaglutide was weighed, dissolved in 1500 mL of a mixed solvent of 25% acetonitrile and 75% water, and purified using Waters 2545 RP-HPLC system with a wavelength of 218 nm, a chromatographic column of 50 x 250 mm reverse phase C18 column, a column temperature of 45°C, and a conventional 0.1% TFA/acetonitrile mobile phase. Target peak components were collected to obtain refined peptides with a purity of more than 98.5%. The refined peptide solution was transformed into salts using Waters 2545 RP-HPLC system with a chromatographic column of 50 x 250 mm reverse phase C18 column and 0.1% hydrochloric acid/acetonitrile as a mobile phase. Target peak components were collected, concentrated under reduced pressure, and lyophilized to obtain 10.86 g of refined Semaglutide hydrochloride with a HPLC purity of 98.98%.

### Example 16: Cleavage and purification of Semaglutide

180 g of Semaglutide Wang resin obtained in Example 11 was taken and added into a 3 L reaction kettle. 1.8 L cleaving agent was formulated in a volume ratio of TFA:thioanisole:water:Phenol:EDT=82.5:5:5:5:2.5, and pre-frozen for 2 h, and then poured into the 3 L reaction kettle, allowing to react for 2 h at the room temperature. After the reaction was completed, the resin was filtered off and the filtrate was collected. The resin was washed with a small amount of TFA, and the filtrates were combined. The filtrate was added to 40 L of ice-cold anhydrous diethyl ether, precipitated, centrifuged, washed with anhydrous diethyl ether, and dried under vacuum to obtain 101 g of crude Semaglutide with a purity of 62.6%. MALDI-TOF: (M+H)⁺=4113.5, as shown in Figure 4.

49.5 g of crude Semaglutide was weighed, dissolved in 1500 mL of a mixed solvent of 25% acetonitrile and 75% water, and purified using Waters 2545 RP-HPLC system with a wavelength of 218 nm, a chromatographic column of 50 x 250 mm reverse phase C18 column, a column temperature of 45°C, and a conventional 0.1% TFA/acetonitrile mobile phase. Target peak components were collected to obtain refined peptides with a purity of more than 98.5%. The refined peptide solution was transformed into salts using Waters 2545 RP-HPLC system with a chromatographic column of 50 x 250 mm reverse phase C18 column and 0.1% hydrochloric acid/acetonitrile as a mobile phase. Target peak components were collected, concentrated under reduced pressure, and lyophilized to obtain 11.12 g of refined Semaglutide hydrochloride with a HPLC purity of 98.86%.

### Example 17: Cleavage and purification of Semaglutide

203 g of Semaglutide 2-CTC resin obtained in Example 13 was taken and added into a 3 L reaction kettle. 1.8 L cleaving agent was formulated in a volume ratio of TFA:thioanisole:PhOMe:TIS:EDT=82.5:5:5:5:2.5, and pre-frozen for 2 h, and then poured into the 3 L reaction kettle, allowing to react for 2 h at the room temperature. After the reaction was completed, the resin was filtered off and the filtrate was collected. The resin was washed with a small amount of TFA, and the filtrates were combined. The filtrate was added to 40 L of ice-cold anhydrous diethyl ether, precipitated, centrifuged, washed with anhydrous diethyl ether, and dried under vacuum to obtain 85 g of crude Semaglutide with a purity of 65.3%. MALDI-TOF: (M+H)⁺=4113.8, as shown in Figure 7.

40.6 g of crude Semaglutide was weighed, dissolved in 1500 mL of a mixed solvent of 25% acetonitrile and 75% water, and purified using Waters 2545 RP-HPLC system with a wavelength of 218 nm, a chromatographic column of 50 x 250 mm reverse phase C18 column, a column temperature of 45°C, and NH₄Ac acetonitrile as a mobile phase system. Target peak components were collected to obtain refined peptides with a purity of more than 98.5%. The refined peptide solution was transformed into salts using Waters 2545 RP-HPLC system with a chromatographic column of 50 x 250 mm reverse phase C18 column and 0.1% acetic acid/acetonitrile as a mobile phase. Target peak components were collected, concentrated under reduced pressure, and lyophilized to obtain 8.36 g of refined Semaglutide acetate with a HPLC purity of 99.02%.

### Example 18: Cleavage and purification of Semaglutide

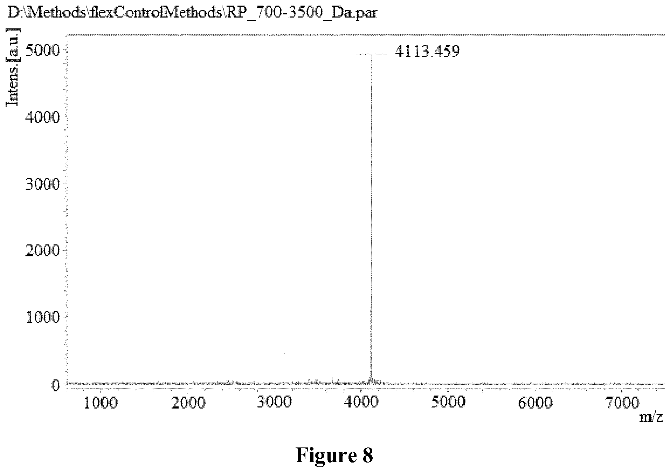

220 g of Semaglutide 2-CTC resin obtained in Example 14 was taken and added into a 3 L reaction kettle. 2.2 L cleaving agent was formulated in a volume ratio of TFA:H₂O:EDT= 90:5:5, and pre-frozen for 2 h, and then poured into the 3 L reaction kettle, allowing to react for 2 h at the room temperature. After the reaction was completed, the resin was filtered off and the filtrate was collected. The resin was washed with a small amount of TFA, and the filtrates were combined. The filtrate was added to 40 L of ice-cold anhydrous diethyl ether, precipitated, centrifuged, washed with anhydrous diethyl ether, and dried under vacuum to obtain 79 g of crude Semaglutide with a purity of 72.4%. MALDI-TOF: (M+H)⁺=4113.5, as shown in Figure 8.

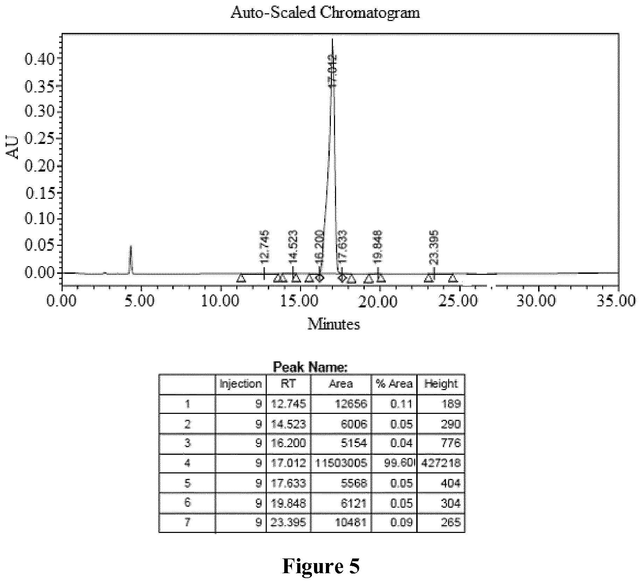

34.8 g of crude Semaglutide was weighed, dissolved in 1500 mL of a mixed solvent of 25% acetonitrile and 75% water, and purified using Waters 2545 RP-HPLC system with a wavelength of 218 nm, a chromatographic column of 50 x 250 mm reverse phase C18 column, a column temperature of 45°C, and NaClO4 and acetonitrile as a mobile phase system. Target peak components were collected to obtain refined peptides with a purity of more than 98.5%. The refined peptide solution was transformed into salts using Waters 2545 RP-HPLC system with a chromatographic column of 50 x 250 mm reverse phase C18 column and 0.1% trifluoroacetic acid/acetonitrile as a mobile phase. Target peak components were collected, concentrated under reduced pressure, and lyophilized to obtain 11.98 g of refined Semaglutide trifluoroacetate with a HPLC purity of 99.60%, as shown in Figure 5.

## Claims

1. A preparation method of Semaglutide, comprising the steps of:
step 1: coupling Gly to a resin by solid phase synthesis to obtain Gly-resin; and
step 2: successively coupling the Gly-resin prepared in step 1 to an amino acid according to the sequence of Semaglutide by sequential coupling; cleaving, and purifying;
wherein Fmoc-Lys(Alloc)-OH is used as a raw material of Lys²⁶;
H-His⁷ is synthesized using Boc-His(Trt)-OH or Boc-His(Boc)-OH.DCHA as a raw material; and
the Lys²⁶ side chain is prepared by fragmental synthesis, and a fully-protected fragment of Fmoc-PEG-PEG-γ-Glu-octadecanedioic acid used in the fragmental synthesis of the Lys²⁶ side chain is prepared by successively coupling a chloride resin to Fmoc-PEG-OH, Fmoc-PEG-OH, Fmoc-Glu(OH)-OtBu and octadecanedioic acid mono-tert-butyl ester, cleaving, and recrystallizing to obtain PEG-PEG-γ-Glu-Octadecanedioic Acid mono-tert-butyl ester.

2. The preparation method according to claim 1, wherein the resin in step 1 is selected from wang resin or 2-CTC resin;
wherein the wang resin has a degree of substitution of 0.1-0.5 mmol/g;
the 2-CTC resin has a degree of substitution of 0.2-0.6 mmol/g;
the wang resin is coupled by DIC+A+DMAP, wherein A is HOBt or HOAt; and
the 2-CTC resin is coupled by DiPEA method.

3. The preparation method according to claim 1 or 2, wherein the coupling in step 2 is performed by condensing by DIC+A or B+A+C method until the resin is detected to be transparent with ninhydrin, wherein A is chosen from HOBt or HOAt, B is chosen from HBTU, HATU, TBTU, PyAOP, or PyBOP, C is chosen from DIPEA or TMP; and solvent is chosen from one or a mixture of two or more of DMF, CH₂Cl₂, NMP, or DMSO.

4. The preparation method according to any one of claims 1 to 3, wherein the coupling in the fragmental synthesis of the Lys²⁶ side chain is performed by condensing with DIC+A or B+A+C until the resin is detected to be transparent with ninhydrin, wherein A is chosen from HOBt or HOAt, B is chosen from HBTU, HATU, TBTU, PyAOP, or PyBOP, C is chosen from DIPEA or TMP; and solvent is chosen from one or a mixture of two or more of DMF, CH₂Cl₂, NMP, or DMSO.

5. The preparation method according to any one of claims 1 to 4, wherein in the fragmental synthesis of the Lys²⁶ side chain, the cleaving is performed with 1-2% TFA/CH₂Cl₂ (V/V) or 20% TFE/CH₂Cl₂ (V/V).

6. The preparation method according to any one of claims 1 to 5, wherein in the fragmental synthesis of the Lys²⁶ side chain, the recrystallizing is performed with one or a mixture of two or more of Et₂O, CH₂Cl₂, CHCl₃, MeOH, CH₃CN, THF, EtOAc, EtOH, Toluene, Hexane, or Acetone.

7. The preparation method according to any one of claims 1 to 6, wherein Alloc in the Fmoc-Lys(Alloc)-OH is removed twice with either 5-10 equivalents of morpholine or 5-10 equivalents of phenylsilane, and 0.1-0.3 equivalents of Pd(PPh₃)₄ for 10-30 min each time, wherein CH₂Cl₂ is used as a solvent in the removal.

8. The preparation method according to any one of claims 1 to 7, wherein for Boc-His(Trt)-OH, the coupling is performed by using a B+A+C system; and for Boc-His(Boc)-OH.DCHA, the coupling is performed by using a HOAT + HATU + TMP system;
wherein A is chosen from HOBt or HOAt, B is chosen from HBTU, HATU, TBTU, PyAOP or PyBOP, C is chosen from DIPEA or TMP; and solvent is chosen from one or a mixture of two or more of DMF, CH₂Cl₂, NMP or DMSO.

9. The preparation method according to any one of claims 1 to 8, wherein the cleaving in step 2 is performed with an reagent comprising one or a mixture of two or more of TFA, PhSMe, PhOMe, EDT, H₂O, TIS or PhOH;
wherein TFA, PhSMe, PhOMe, EDT, H₂O, TIS and PhOH are in a volume ratio of 80-90:0-5:0-3:0-5:0-5:0-2:0-5.

## Patentansprüche

1. Verfahren zur Herstellung von Semaglutid, umfassend die folgenden Schritte:
Schritt 1: Koppeln von Gly an ein Harz durch Festphasensynthese, um Gly-Harz zu erhalten; und
Schritt 2: sukzessives Koppeln des in Schritt 1 hergestellten Gly-Harzes an eine Aminosäure gemäß der Sequenz von Semaglutid durch sequentielle Kopplung; Spalten und Reinigen;
wobei Fmoc-Lys (Alloc)-OH als ein Rohmaterial von Lys²⁶ verwendet wird;
H-His⁷ unter Verwendung von Boc-His(Trt)-OH oder Boc-His(Boc) -OH.DCHA als ein Rohmaterial synthetisiert wird; und
die Lys²⁶-Seitenkette durch Fragmentensynthese hergestellt wird, und ein vollständig geschütztes Fragment von Fmoc-PEG-PEG-γ-Glu-octadecandionsäure, das in der Fragmentensynthese der Lys²⁶-Seitenkette verwendet wird, durch sukzessives Koppeln eines Chloridharzes an Fmoc-PEG-OH, Fmoc-PEG-OH, Fmoc-Glu(OH)-OtBu und Octadecandionsäure-mono-tert-butylester, Spalten und Rekristallisieren hergestellt wird, um PEG-PEG-γ-Glu-Octadecandionsäure-mono-tert-butylester zu erhalten.

2. Verfahren zur Herstellung nach Anspruch 1, wobei das Harz in Schritt 1 ausgewählt ist aus Wang-Harz oder 2-CTC-Harz;
wobei das Wang-Harz einen Substitutionsgrad von 0,1 - 0,5 mmol/g aufweist;
das 2-CTC-Harz einen Substitutionsgrad von 0,2 - 0,6 mmol/g aufweist;
das Wang-Harz durch DIC+A+DMAP gekoppelt ist, wobei A HOBt oder HOAt ist; und das 2-CTC-Harz durch das DiPEA-Verfahren gekoppelt wird.

3. Verfahren zur Herstellung nach einem der Ansprüche 1 oder 2, wobei die Kopplung in Schritt 2 durch Kondensieren durch ein DIC+A- oder B+A+C-Verfahren durchgeführt wird, bis nachgewiesen wird, dass das Harz mit Ninhydrin transparent ist, wobei A ausgewählt ist aus HOBt oder HOAt, B ausgewählt ist aus HBTU, HATU, TBTU, PyAOP oder PyBOP, C ausgewählt ist aus DIPEA oder TMP; und das Lösungsmittel ausgewählt ist aus einem oder einem Gemisch von zwei oder mehr DMF, CH₂Cl₂, NMP oder DMSO.

4. Verfahren zur Herstellung nach einem der Ansprüche 1 bis 3, wobei die Kopplung bei der Fragmentensynthese der Lys²⁶-Seitenkette durch Kondensation mit DIC+A oder
B+A+C durchgeführt wird, bis nachgewiesen wird, dass das Harz mit Ninhydrin transparent ist, wobei A ausgewählt ist aus HOBt oder HOAt, B ausgewählt ist aus HBTU, HATU, TBTU, PyAOP oder PyBOP, C ausgewählt ist aus DIPEA oder TMP; und das Lösungsmittel ausgewählt ist aus einem oder einem Gemisch von zwei oder mehr DMF, CH₂Cl₂, NMP oder DMSO.

5. Verfahren zur Herstellung nach einem der Ansprüche 1 bis 4, wobei bei der Fragmentensynthese der Lys²⁶-Seitenkette die Spaltung mit 1-2 % TFA/CH₂Cl₂ (V/V) oder 20 % TFE/CH₂Cl₂ (V/V) durchgeführt wird.

6. Verfahren zur Herstellung nach einem der Ansprüche 1 bis 5, wobei bei der Fragmentensynthese der Lys²⁶-Seitenkette die Rekristallisation mit einem oder einem Gemisch von zwei oder mehr Et₂O, CH₂Cl₂, CHCl₃, MeOH, CH₃CN, THF, EtOAc, EtOH, Toluol, Hexan oder Aceton durchgeführt wird.

7. Verfahren zur Herstellung nach einem der Ansprüche 1 bis 6, wobei Alloc in dem Fmoc-Lys(Alloc)-OH zweimal mit entweder 5-10 Äquivalenten von Morpholin oder 5-10 Äquivalenten von Phenylsilan und 0,1-0,3 Äquivalenten von Pd(PPh₃)₄ jedes Mal für 10 bis 30 Minuten entfernt wird, wobei CH₂Cl₂ als ein Lösungsmittel bei der Entfernung verwendet wird.

8. Verfahren zur Herstellung nach einem der Ansprüche 1 bis 7, wobei für Boc-His(Trt)-OH die Kopplung unter Verwendung eines B+A+C-Systems durchgeführt wird; und für Boc-His(Boc)-OH.DCHA die Kopplung unter Verwendung eines HOAT + HATU + TMP-Systems durchgeführt wird;
wobei A ausgewählt ist aus HOBt oder HOAt, B ausgewählt ist aus HBTU, HATU, TBTU, PyAOP oder PyBOP, C ausgewählt ist aus DIPEA oder TMP; und das Lösungsmittel ausgewählt ist aus einem oder einem Gemisch von zwei oder mehr DMF, CH₂Cl₂, NMP oder DMSO.

9. Verfahren zur Herstellung nach einem der Ansprüche 1 bis 8, wobei das Spalten in Schritt 2 mit einem Reagenz durchgeführt wird, das ein oder ein Gemisch von zwei oder mehr von TFA, PhSMe, PhOMe, EDT, H₂O, TIS or PhOH umfasst;
wobei TFA, PhSMe, PhOMe, EDT, H₂O, TIS und PhOH in einem Volumenverhältnis von 80-90: 0-5: 0-3: 0-5: 0-5: 0-2: 0-5 vorliegen.

## Revendications

1. Procédé de préparation du Sémaglutide, comprenant les étapes de :
étape 1 : couplage de Gly à une résine par une synthèse en phase solide pour obtenir une Gly-résine ; et
étape 2 : couplage successif de la Gly-résine préparée dans l'étape 1 à un acide aminé selon la séquence du Sémaglutide par couplage séquentiel ; clivage, et purification ;
dans lequel du Fmoc-Lys(Alloc)-OH est utilisé comme une matière brute de Lys²⁶ ;
H-His⁷ est synthétisé en utilisant du Boc-His(Trt)-OH ou du Boc-His(Boc)-OH.DCHA comme une matière brute ; et
la chaîne latérale de Lys²⁶ est préparée par synthèse fragmentaire, et un fragment entièrement protégé de Fmoc-PEG-PEG-γ-Glu-acide octadécanedioïque utilisé dans la synthèse fragmentaire de la chaîne latérale de Lys²⁶ est préparé par le couplage successif d'une résine de chlorure à du Fmoc-PEG-OH, du Fmoc-PEG-OH, du Fmoc-Glu(OH)-OtBu et un ester mono-tert-buytlique d'acide octadécanedioïque, clivage et recristallisation pour obtenir un PEG-PEG-γ-Glu-ester mono-tert-butylique d'acide acide octadécanedioïque.

2. Procédé de préparation selon la revendication 1, dans lequel la résine dans l'étape 1 est choisie parmi de la résine de Wang ou de la résine 2-CTC ;
dans lequel la résine de Wang a un degré de substitution de 0,1 à 0,5 mmol/g ;
la résine 2-CTC a un degré de substitution de 0,2 à 0,6 mmol/g ;
la résine de Wang est couplée par DIC+A+DMAP, dans lequel A est du HOBt ou du HOAt ; et la résine 2-CTC est couplée par un procédé DiPEA.

3. Procédé de préparation selon la revendication 1 ou 2, dans lequel le couplage dans l'étape 2 est réalisé par condensation par un procédé DIC+A ou B+A+C jusqu'à ce que la résine soit détectée comme étant transparente avec de la ninhydrine, dans lequel A est choisi parmi du HOBt ou du HOAt, B est choisi parmi du HBTU, du HATU, du TBTU, du PyAOP, ou du PyBOP, C est choisi parmi de la DIPEA ou du TMP ; et le solvant est choisi parmi un ou un mélange de deux ou plus de DMF, CH₂Cl₂, NMP, ou DMSO.

4. Procédé de préparation selon l'une quelconque des revendications 1 à 3, dans lequel le couplage dans la synthèse fragmentaire de la chaîne latérale de Lys²⁶ est réalisé par condensation avec DIC+A ou B+A+C jusqu'à ce que la résine soit détectée comme étant transparente avec de la ninhydrine, dans lequel A est choisi parmi du HOBt ou du HOAt, B est choisi parmi du HBTU, du HATU, du TBTU, du PyAOP, ou du PyBOP, C est choisi parmi de la DIPEA ou du TMP ; et le solvant est choisi parmi un ou un mélange de deux ou plus de DMF, CH₂Cl₂, NMP, ou DMSO.

5. Procédé de préparation selon l'une quelconque des revendications 1 à 4, dans lequel dans la synthèse fragmentaire de la chaîne latérale de Lys²⁶, le clivage est réalisé avec 1 à 2 % de TFA/CH₂Cl₂ (V/V) ou 20 % de TFE/CH₂Cl₂ (V/V).

6. Procédé de préparation selon l'une quelconque des revendications 1 à 5, dans lequel dans la synthèse fragmentaire de la chaîne latérale de Lys²⁶, la recristallisation est réalisée avec un ou un mélange de deux ou plus de Et₂O, CH₂Cl₂, CHCl₃, MeOH, CH₃CN, THF, EtOAc, EtOH, Toluène, Hexane, ou Acétone.

7. Procédé de préparation selon l'une quelconque des revendications 1 à 6, dans lequel l'Alloc dans le Fmoc-Lys(Alloc)-OH est éliminé deux fois avec soit 5 à 10 équivalents de morpholine ou 5 à 10 équivalents de phénylsilane, et 0,1 à 0,3 équivalent de Pd(PPh₃)₄ pendant 10 à 30 min chaque fois, dans lequel du CH₂Cl₂ est utilisé comme un solvant dans l'élimination.

8. Procédé de préparation selon l'une quelconque des revendications 1 à 7, dans lequel pour le Boc-His(Trt)-OH, le couplage est réalisé en utilisant un système B+A+C ; et pour Boc-His(Boc)- OH.DCHA, le couplage est utilisé en utilisant un système HOAT + HATU + TMP ;
dans lequel A est choisi parmi du HOBt ou du HOAt, B est choisi parmi du HBTU, du HATU, du TBTU, du PyAOP ou du PyBOP, C est choisi parmi de la DIPEA ou du TMP ; et le solvant est choisi parmi un ou un mélange de deux ou plus de DMF, CH₂Cl₂, NMP ou DMSO.

9. Procédé de préparation selon l'une quelconque des revendications 1 à 8, dans lequel le clivage dans l'étape 2 est réalisé avec un réactif comprenant un ou un mélange de deux ou plus de TFA, PhSMe, PhOMe, EDT, H₂O, TIS ou PhOH ;
dans lequel les TFA, PhSMe, PhOMe, EDT, H₂O, TIS et PhOH sont dans un rapport volumique de 80 à 90/0 à 5/0 à 3/0 à 5/0 à 5/0 à 2/0 à 5.
